# EUROPEAN PATENT SPECIFICATION

(11) **EP 1 729 867 B1**
(45) Date of publication and mention of the grant of the patent: **27.10.2010**
(21) Application number: 05722292.9
(22) Date of filing: 31.03.2005
(51) Int. Cl.: B01D 15/08, C07K 1/36

(54) **A METHOD FOR CHROMATOGRAPHIC PURIFICATION**
VERFAHREN ZUR CHROMATOGRAPHISCHEN REINIGUNG
PROCEDE DE PURIFICATION CHROMATOGRAPHIQUE

(30) Priority: 02.04.2004 SE 0400886
(43) Date of publication of application: 13.12.2006
(73) Proprietor: GE Healthcare Bio-Sciences AB, 75451 Uppsala (SE)
(72) Inventor: VAN ALSTINE, James Amersham Biosciences AB, S-751 84 Uppsala (SE); HOUSHMAND, Hamid Amersham Biosciences AB, S-751 84 Uppsala (SE); LJUNGLÖF, Anders Amersham Biosciences AB, S-751 84 Uppsala (SE); ÅBERG, Per-Mikael Amersham Biosciences AB, S-751 84 Uppsala (SE)
(74) Representative: Kilander, Ebba Annika
(86) International application number: PCT/SE2005/000467
(87) International publication number: WO 2005/094960

(56) References cited:
- EP-A2- 1 319 711
- US-A- 3 869 436
- US-A- 4 683 294
- US-A- 5 151 358
- US-B1- 6 383 393
- SHIBUSAWA Y. ET AL.: 'Purification of lactic acid dehydrogenase from bovine heart crude extract by counter-current chromatography.' JOURNAL OF CHROMATOGRAPHY B. vol. 696, no. 1, 15 August 1997, pages 25 - 31, XP004087048
- GEHRKE S. ET AL.: 'Protein Sorption and Revory by Hydrogels Using Principles of Aqueous Two-Phase Extraction.' BIOTECHNOLOGY AND BIOENGINEERING vol. 58, no. 4, 20 May 1998, pages 416 - 427, XP008071329

## Description

### Technical field

The present invention relates to the field of biotechnology, and more specifically to the purification of biological compounds, such as proteins, antibodies and the like. Thus, the present invention relates to a method of liquid chromatography for the purification of a target compound from one or more other components of a liquid as well as to a kit that enables performing such purification.

### Background

Biotechnological methods are used to an increasing extent in the production of proteins, peptides, nucleic acids and other biological compounds, for research purposes as well as in order to prepare novel kinds of drugs. Due to its versatility and sensitivity to the compounds, chromatography is often the preferred purification method in this context. The term chromatography embraces a family of closely related separation methods, which are all based on the principle that two mutually immiscible phases are brought into contact. More specifically, the target compound is introduced into a mobile phase, which is contacted with a stationary phase. The target compound will then undergo a series of interactions between the stationary and mobile phases as it is being carried through the system by the mobile phase. The interactions exploit differences in the physical or chemical properties of the components in the sample.

In liquid-solid chromatography, commonly referred to simply as liquid chromatography, the target compound is present in a liquid together with one or more contaminants or undesired substances. Said liquid is contacted with a stationary phase, known as a matrix, which is commonly comprised of either a collection of homogenous, porous or non-porous particles or a monolith of organic or inorganic origin. The properties of the separation matrix will in large decide the efficiency obtained when used in a separation process, such as chromatography. Usually, a separation matrix is comprised of a carrier to which groups capable of interaction with the target and known as ligands have been coupled. Thus, the ligands will impart to the carrier the ability to effect the separation, identification, and/or purification of molecules of interest. A review of various chromatography methods useful for protein purification is provided e.g. in Protein Purification - Principles, High Resolution Methods and Applications (J.-C. Janson and L. Rydén, 1989 VCH Publishers, Inc.).

Thus, in brief, ion-exchange chromatography is a well-known method of fractionation frequently used for isolation of biological compounds, such as proteins. The basis for the ion-exchange chromatography process is the competitive binding of ions of one kind, such as proteins, for ions of another kind, such as salt ions, to an oppositely charged matrix known as the ion-exchanger. The interaction between the proteins and the ion-exchanger depends on several factors, such as net charge and surface charge distribution of the protein, the ionic strength and the nature of the particular ions in the solvent, the proton activity (pH) etc. More specifically, the adsorption stage of ion-exchange chromatography usually occurs in dilute salt solutions, i.e. solutions of relatively low conductivity, where charge-charge interactions are strong; while the elution stage occurs in solutions of higher conductivity. Differential elution of mixtures of substances often requires elution with gradients of increasing salt concentration. Furthermore, if two ion-exchange chromatography steps are combined, since a high conducting solution is used for elution of the first column, a dilution step will be required before the elute of interest can be loaded onto the subsequent ion-exchanger. For this reason a dilution step is also required before the first column if the mobile phase is a fermentation broth comprising e.g. recombinant proteins. However, the need for dilution will increase the volumes and sizes of equipment required, increasing the cost of the process and making it more cumbersome to work.

Affinity chromatography owes its name to the exploitation of various affinities for adsorption to a solid phase. Useful in affinity chromatography are e.g. interactions between a protein and low molecular weight substances; between bioinformative molecules such as hormones and receptors; and between biopolymers, in particular proteins. Examples can be found in all areas of structural and physiological biochemistry such as in multimolecular assemblies, effector-receptor interactions, DNA-protein interactions and antigen-antibody binding. Protein A and Protein G affinity chromatography are today maybe the best established methods for isolation and purification of immunoglobulins, particularly for isolation of monoclonal antibodies, mainly due to the ease of use, the outstanding selectivity and the high purity obtained. In many cases elution from an affinity matrix requires increasing solution conductivity or altering pH so as to reduce charge-charge or other interactions. However, for reasons discussed above, in a multi-step chromatography procedure conventional affinity chromatography using salt gradient elution will require a subsequent dilution step before the next step of ion-exchange chromatography.

In hydrophobic chromatography (HIC), a hydrophobic interaction is utilised for the binding of target compounds, such as proteins, to matrices that present hydrophobic ligands. More specifically, the surfaces of globular proteins typically have extensive hydrophobic patches, which bind to hydrophobic ligands on matrices. Thus, HIC adsorption is typically done in high conductivity solutions. The release of target compounds is commonly obtained by running a gradient of decreasing salt concentration and utilising the differences in the strength of interaction between target and ligands. In some cases, a decrease of the solvent polarity is also needed for elution. Thus, a bound compound may be released from the matrix by lowering the concentration of lyotropic salt in a continuous or stepwise gradient. If a highly pure product is the object, it is often recommended to combine the hydrophobic chromatography with a further step. However, if HIC is to be combined with a preceding ion-exchange chromatography step, to obtain the required conductivity in the mobile phase before loading thereof onto the HIC column, salt will need to be added.

Thus, at present, most biomolecules, such as proteins, are purified by processes involving a series of purification steps often involving one or more chromatographic steps. However, the cost and efficiency of many processes are dramatically affected by dilutions, pH changes, and other operations necessary to interface various separation steps. The above suggests that if one could operate multi-step chromatographic processes using solutions of more similar salt composition or conductivities, i.e. without extensive salt change or dilution, it would make such processes more effective. Such a multi-step chromatographic process would be especially advantageous if it also involved increased dynamic capacities of the matrices.

Another combination of two or more purification steps is disclosed in US 3,869,436 (Statens Bakteriologiska Laboratorium), which relates to a method of fractionating plasma proteins using ion-exchange chromatography. More specifically, US 3,869,436 discloses a method wherein in brief, the globulins in the plasma are precipitated with poly(ethylene glycol); all of the precipitate is centrifuged out of the remaining solution; the precipitate is dissolved in sodium acetacetic acid; adsorbing the globulins from the dissolved precipitate on a cation-exchanger; precipitating the eluate with poly(ethylene glycol) (PEG); dissolving the precipitate in phosphate buffer; and adsorbing the globulins from the dissolved precipitate on an anion-exchanger. PEG 6000 is used, at a final concentration of about 13%, to obtain the desired precipitation, which is also affected by ionic strength, protein concentration, temperature and pH. According to US 3,869,436, an advantage of precipitation with organic solvents, compared to neutral salts as earlier suggested, is that their volatility allows them to be easily removed by means of freeze-vacuum drying.

Poly(ethylene glycol) (PEG) has also been suggested as a mobile phase modifier in hydrophobic interaction chromatography (HIC) for purification of various proteins including monoclonal antibodies (Pete Gagnon, Purification Tools for Monoclonal Antibodies, Ch. 6, pp. 103-126 in Validated Biosystems, Tucson, AZ, 1996, ISBN 0-9653515-9-9). PEG has also been advocated as a mobile phase additive for hydrophilic interaction chromatography (HILIC), which is a chromatographic method similar to HIC, but employing more hydrophilic matrices and relying more on cohydration than hydrophobic interactions (Pete Gagnon, Purification Tools for Monoclonal Antibodies, Ch. 8, pp. 138-143, Validated Biosystems, Tucson, AZ, 1996, ISBN 0-9653515-9-9). The PEG is run in a gradient, as the conventional use of salt. The apparent mechanism of the salt and PEG is to bind mobile phase water, which favours protein cohydration interaction to the matrix where more hydrating water is retained.

In order to improve binding efficiencies in affinity chromatography, P. Gagnon (Pete Gagnon, Purification Tools for Monoclonal Antibodies, Ch. 9, pp. 159, Validated Biosystems, Tucson, AZ, 1996, ISBN 0-9653515-9-9) explored some of the primary operating variables in the Protein A binding of IgG, which binding has been documented as being predominantly hydrophobic. To characterise the basic means by which Protein A binding can be optimised, surface tension, volumetric exclusion and dielectric constant were evaluated individually and in limited combinations by adding betaine or poly(ethylene glycol) (PEG) to the binding buffer. It was concluded that surface tension and volumetric exclusion both enhanced binding with roughly equal effectivity, while the dielectric constant had no apparent effect. It was also noted that poly(ethylene glycol) (PEG) produced a response similar to precipitating salts.

Finally, poly(ethylene glycol) (PEG) has been suggested as an additive to the load of fermentation broths of high conductivity levels to enhance the dynamic binding capacity of ion-exchange chromatography (Chavez et al: "Increased Dynamic Binding Capacity in Ion-exchange Chromatography by Addition of Poly(ethylene glycol)", Abstract from Recovery of Biological Products XI meeting, Banff, Canada, September 14-19, 2003). Model proteins were tested on the matrices Fractogel™ SO₃ and Fractogel™ TMAE (EM Industries) and SP Sepharose™ and Q Sepharose™ FF (Amersham Biosciences, Uppsala, Sweden). Fractogel™ comprises a carrier of cross-linked polymethacrylate, with a pore size of about 800 Å, wherein to which the functional ion-exchanger groups are bonded via linear polymer chains. Sepharose™ is a porous cross-linked agarose carrier to which S or Q groups have been covalently coupled. The proposed mechanism is that the hydrated PEG disrupts the protein and resin hydration shells. Consequently, it becomes favourable for the protein to bind to the resin, even under high conductivity conditions. The disclosed effect is most pronounced with both higher PEG MW, such as above 3350, and protein MW. Not surprisingly, this observation is analogous to PEG precipitation properties.

However, in spite of the above work related to mobile phase PEG enhancing the capacity of single matrix, there is still need for the deeper scientific appreciation which allows adaptation of such effects to improve the efficiency of multi-step bioseparation processes.

### Brief description of the present invention

The present invention relates to enhancing the binding capacity in chromatographic processes. One aspect of the present invention is a method of isolating one or more target compounds by chromatography comprising a first and a second chromatographic step, wherein the dynamic binding capacity of a chromatography matrix is enhanced. This can be achieved by using a combination of two or more steps of affinity chromatography and ion-exchange chromatography and/or hydrophobic interaction chromatography, wherein a non-ionic polyether, such as poly(ethylene glycol) (PEG), is present during the adsorption of target compound to the affinity step.

Another aspect of the present invention is a multi step chromatographic method, wherein the efficiency is improved by reducing dilution steps or volumes.

Thus, a specific aspect of the invention is a chromatographic method comprising two or more consecutive ion-exchange chromatography steps, wherein the need to dilute the mobile phase between said steps is reduced or even eliminated.

Another aspect of the invention is a chromatographic method comprising an affinity step followed by an ion-exchange chromatography step, wherein there is no need of dilution of the eluate from the first step before loading thereof onto the second step.

Yet another aspect of the invention is a chromatographic method comprising an ion-exchange chromatography step followed by a hydrophobic interaction chromatography step, wherein the need of adding any salt to the eluate from the first step before loading thereof onto the second step is reduced or avoided.

An aspect of the present invention is a method of purification of biological compounds such as proteins, wherein a non-ionic polyether, such as poly(ethylene glycol) (PEG) is added to the mobile phase before the adsorption step, but is not included in the elution step, which method avoids the need of clearance of residual PEG from the product.

Further aspects and advantages of the present invention will appear from the detailed description that follows.

### Brief description of the drawings

Figure 1 compares a three-step protein purification process according to the invention in the presence (right) and absence (left) of a non-ionic polyether by presenting a comparison of hypothetical affinity capture, IEX purification and HIC polishing steps. Note how the latter avoids costly dilution or desalting steps, and switching between high and low salt buffers. Related pH adjustment steps are comparable and not shown.
Figure 2 compares in a flow chart similar to Figure 1 a three-step protein purification process according to the invention in the presence (right) and absence (left) of a non-ionic polyether: affinity chromatography, ion-exchange chromatography and ion-exchange chromatography.
Figure 3 shows the effect of different lyotropic series salts on the ability of mobile phase added non-ionic polymer to enhance the dynamic capacity (Q_{B10%}) of bovine serum albumin protein on both two commercially available chromatography matrices. The effect increases in the normal ion-exchange (IEX) order NaI < NaCl < NaAc = NaF.
Figure 4 is a chromatogram showing frontal analysis using BSA with PEG added to the buffer.
Figure 5 shows in a diagram how the BSA dynamic capacity of a commercial cation-exchanger for BSA in increases with added mobile phase concentration (addition at adsorption) of PEG.
Figure 6 shows how mobile phase added PEG, i.e. a non-ionic polyether added before the adsorption, increases the dynamic capacity of two commercial cation-exchangers.
Figure 7 shows how mobile phase added PEG enhances the dynamic capacity of a commercial cation-exchanger matrix for both polyclonal and monoclonal antibody samples, with the effect more pronounced in buffers with higher salt concentration.
Figure 8 shows how mobile phase added PEG is able to also enhance the dynamic capacity of a commercially available affinity matrix for polyclonal antibody.

### Definitions

The term "eluent" means a liquid such as a buffer which due to its chemical composition or physical properties is capable of releasing an adsorbed compound from a chromatography matrix.

The term a "biological compound" means any biological molecule or compound, such as proteins, peptides, amino acids, cells, nucleic acids etc.

The term "affinity" chromatography and matrix refers the binding of a biological molecule, or part of a molecule, to a specific ligand based on a biological "lock/key" mechanism. Common examples of affinity binding is the binding of an antigen to a specific antibody; the binding of an enzyme to a specific receptor etc.

The term "mobile phase" means the buffer used in the adsorption step.

The term "matrix" means herein a porous or non-porous carrier to which ligands have been immobilised.

The term "ligands" means herein any compound that comprises functional groups capable of interacting with a target compound.

The term a "charged ligand" means a ligand that comprises groups, which in their charged state are capable of electrostatic interaction with a target compound of opposite charge.

The term "carrier surfaces" includes the external surface and, in the case of a porous carrier, the pore surfaces.

The term an "antibody compound" embraces functional antibody fragments and fusion proteins comprising antibodies. In this context, a "functional" antibody fragment means a fragment, which has retained essentially the original binding properties of the antibody.

### Detailed description of the invention

The present invention relates to purification of target compounds, such as antibodies and antibody compounds, by chromatography. More specifically, the present inventors have found that the presence of non-ionic polyether in the mobile phase, often denoted the adsorption buffer, results in an unexpected binding capacity, especially if two or more chromatographic steps using such non-ionic polyether are combined.

It has also been shown that the present invention is efficient even at high salt concentrations, which is a common case if the target compounds have been produced by fermentation of target compound-producing cells. Thus, the present invention may reduce the need to dilute prior to adsorption and allow for enhanced process efficiency.

Thus, in a first aspect, the present invention relates to a method of isolating one or more target compounds from other component(s) of a liquid by at least two chromatographic steps, which method comprises contacting the liquid, in any sequence of order, with an affinity chromatography matrix and an ion-exchange chromatography matrix and/or a hydrophobic interaction chromatography matrix to provide interactions between the target compound and the matrices, wherein the contacting with the affinity matrix takes place in the presence of at least one non-ionic polyether; and obtaining the target compound(s) in a separate fraction from the last chromatographic step. In one embodiment, one or more contacting with chromatography matrix is performed in the presence of a mixture of two or more non-ionic polyethers.

Preferably, said non-ionic polyether is present in a conventional buffer that provides advantageous adsorption conditions, such as a conventional mobile phase salt buffer solution. The liquid comprising the target compound may be obtained from fermentation as a clarified or unclarified broth, in which case the first step is commonly known as the capture step. In one embodiment, one or more non-ionic polyethers are present during the adsorption of such a capture step. Alternatively, the liquid comprising the target compound is the eluate from a preceding chromatography step.

In one embodiment, the non-ionic polyether, optionally in a buffer, is combined with the liquid comprising the target compound and the resulting mixture will constitute the mobile phase in the subsequent chromatography. In an alternative embodiment, the non-ionic polyether originates from the use of two phase partition prior to the first chromatography step. Conventionally, such two phase partitioning would be followed by removal of the non-ionic polyether, but according to the present invention, the non-ionic polyether may instead be used to enhance the adsorption during chromatography. Thus, the phase comprising non-ionic polyether and target compound can be applied directly onto a chromatography column, optionally with a preceding dilution e.g. with water.

In this context, it is understood that the interactions provided between the target compound and the matrix may be adsorption, i.e. binding of the target, or a retardation of the target compound in relation to other component(s). Thus, in one embodiment, the present invention is a method of isolating one or more target compounds from other component(s) of a liquid by at least two chromatographic steps, which method comprises contacting the liquid, in any sequence of order, with an affinity chromatography matrix and/or an ion-exchange chromatography matrix and/or a hydrophobic interaction chromatography matrix to provide adsorption of the target compound to the matrix, wherein the contacting with at least one of the matrices takes place in the presence of at least one non-ionic polyether; and obtaining the target compound(s) in a separate fraction from the last chromatographic step. In one embodiment, at least one adsorption step is performed in the presence of a mixture of two or more non-ionic polyethers. In order to obtain an isolated target compound, this embodiment is preferably followed by a step of eluting said adsorbed target compound(s). Elution of adsorbed target compounds may be obtained by change of pH and/or salt conditions as compared to the adsorption step, as is well known in this field.

With the exception of the addition of a non-ionic polyether to the mobile phases, the chromatography steps of the present method are performed in accordance with the conventional general operating conditions and well known principles of the field. Conventional chromatography columns are conveniently used for each step, the size of which are adapted for each case, as is the starting materials, buffers, matrices, including the functional groups of the ligands, etc.

In a first embodiment, the present method comprises two or more consecutive ion-exchange chromatography steps. The ion-exchange chromatography may be cation-exchange chromatography or anion-exchange chromatography, depending on the charge of the target compound. As is well known, the charge of a target may be changed by changing the surrounding pH. As is also well known, an ion exchanger may be a strong ion exchanger, which means that it is charged at all pH values; or a weak ion exchanger, which means that it is chargeable by shifting the pH. As will be shown in the experimental part below, the presence of a non-ionic polyether, such as PEG, in the mobile phase applied to the second step enables to perform two consecutive ion-exchange chromatography steps without any intermediate dilution, regardless of the salt gradient used for elution of the first column. Thus, the present invention allows ion-exchange chromatography under conductivities where the target compound would normally elute, such as conductivities that often result from recombinant manufacture of proteins and peptides via fermentation. Hence, since no substantial dilution of a fermentation broth or an eluate from a preceding ion-exchange chromatography step is required, the present invention allows substantial savings as regards both operating volumes and total costs. As is also shown below, adding PEG to the mobile phase of an ion-exchange chromatography step according to the invention was unexpectedly shown to increase the dynamic binding capacity (DBC) of a model protein between four and six times, as compared to conventional ion-exchange chromatography. This effect will herein sometimes be denoted the "PEG effect". Common buffers can be used to modify this effect, such as sodium acetate, and an increase of the salt concentration has been shown by the present inventors to increase the PEG effect. In one embodiment of the present invention, the concentration of PEG in an ion-exchange chromatography step is about 6-10%, such as about 8% PEG.

In an alternative embodiment, the present method comprises an affinity step followed by an ion-exchange chromatography step. Again, this embodiment can be performed without the need to dilute the eluate from the first step before it is loaded onto the subsequent step, allowing substantial savings in process volumes and economy. In the experimental part below, it will be shown how the dynamic binding capacity (DBC) during the affinity step can be increased according to the invention by values such as 1.5 times the corresponding conventional step without addition of non-ionic polyether. An illustrative concentration of non-ionic polyether, such as PEG, in an affinity step is about 4-8%, such as about 6% PEG. In a specific embodiment, the present method is a three step process comprising an affinity chromatography step followed by two ion-exchange chromatography steps.

In another alternative embodiment, the present method comprises an ion-exchange chromatography step followed by a hydrophobic interaction chromatography (HIC) step. In this embodiment, an advantage as compared to the corresponding combination of steps without the addition of non-ionic polyether is that the requirement to increase the conductivity of the mobile phase between the two steps by adding salt will be greatly reduced or even eliminated, since the first step can be performed without the conventional dilution. As the skilled person will realise, this advantage applies in the case of a liquid phase of an originally high conductivity, such as a fermentation broth comprising recombinantly produced proteins or peptides. In a specific embodiment, the process is a three step process, wherein affinity chromatography precedes the ion-exchange chromatography and HIC.

The non-ionic polyether used as an additive in the present method comprises groups that are rich in oxygens. Such target substances are in a preferred embodiment polyethers, such as poly(ethylene glycol) (PEG), polypropylene glycol (PPG), PEG-PPG block copolymers, PEG-PPG copolymers, Pluronic^{™} (BASF) and other PEG-PPG-PEG triblock polymers, ethylhydroxyethylcellulose (EHEC) and similar polymers, polymerised allylglycidyl ether, polymerised phenyl glycidyl ether, plus various surfactants and other compounds which utilise the above mentioned polyethers. In the most advantageous embodiment, the non-ionic polyether is poly(ethylene glycol) (PEG). In the present context, it is to be understood that the term PEG also embraces poly(ethylene glycol) which has been modified without affecting the advantageous properties disclosed herein.

The use of PEG as an additive to the mobile phase in a chromatography step will be described in detail in the Experimental part below. In brief, PEG is available from a number of commercial sources, such as Sigma. Commercially available PEG is denoted due to its molecular weight, such as PEG 4600, PEG 8000 etc. In one embodiment, PEG of a molecular weight in the range of about 1000 to about 20000, such as 5000-15000, e.g. about 10000, is used.

In one embodiment, in the present method, the affinity matrix is comprised of protein ligands immobilised to a carrier. Use of protein or protein fragments as ligands in affinity chromatography is well known in this field, and there are numerous illustrative examples thereof. In one embodiment, the ligands are immunoglobulin-binding proteins, such as protein A or protein G, or immunoglobulin-binding fragments thereof. In an advantageous embodiment, the protein ligands are Protein A. As is well known, Protein A is known as an affinity ligand both in its native form and as a recombinant protein, both of which are included in this embodiment.

The carrier to which the affinity ligands have been immobilised can be of any organic or inorganic material. Thus, the carrier is commonly a solid material, which can be porous or non-porous, as conventionally used in liquid chromatography. In an advantageous embodiment, the carrier is porous. The carrier may be in the form of particles, such as spherical particles, monoliths, strips, membranes, filters, microchips or the like. In one embodiment, the carrier comprises particles which are essentially spherical and in a range from about 1 µm- 1000 µm in diameter, such as 10-500 µm, preferably 40-130 µm, such as about 85 µm. In a specific embodiment, the particles are of a kind specifically designed for expanded bed adsorption, also known as fluidised bed adsorption. In this embodiment, the particles are of a relatively high density, which is commonly obtained by providing them with high density fillers, such as quartz or stainless steel. Expanded bed adsorption separation matrices are commercially available, such as the Streamline™ product group available from Amersham Biosciences, Uppsala, Sweden.

In one embodiment, the carrier is comprised of a cross-linked carbohydrate material, such as agarose, agar, cellulose, dextran, chitosan, konjac, carrageenan, gellan, or alginate. In the most preferred embodiment, the present carrier is porous particles made from cross-linked agarose. The carbohydrate carrier of the invention is easily prepared by the skilled person in this field in accordance with standard methods, such as inverse suspension gelation (S Hjertén: Biochim Biophys Acta 79(2), 393-398 (1964). Alternatively, the carrier is based on a commercially available product, such as Sepharose™ FF (a porous cross-linked agarose gel from Amersham Biosciences AB, Uppsala, Sweden), which product is easily provided with affinity ligands in accordance with standard methods. (See e.g. US 6,399,750 (Pharmacia Biotech) for an illustrative method of binding recombinant Protein A to a carrier, as well as references given therein.)

Alternatively, the carrier of the affinity ligands is comprised of any well known cross-linked synthetic polymer, such as styrene or styrene derivatives, divinylbenzene, acrylamides, acrylate esters, methacrylate esters, vinyl esters, vinyl amides etc. Such polymers are easily produced according to standard methods, see e.g. "Styrene based polymer supports developed by suspension polymerization" (R Arshady: Chimica e L'Industria 70(9), 70-75 (1988)). Alternatively, a commercially available product, such as Source™ (Amersham Biosciences AB, Uppsala, Sweden) can be provided with affinity ligands e.g. by grafting and used in the method according to the invention. (For a review of different principles of grafting, see e.g. P F Rempp, P J Lutz: Comprehensive Polymer Science vol. 6, pp 403-421, Eds. G Allen et al, Oxford 1989. For preparation of synthetic chromatography supports by grafting, see WO 03/046063, Amersham Biosciences AB (Ihre et al)).

In an illustrative embodiment, the affinity step of the present method utilises MabSelect™ (Amersham Biosciences, Uppsala, Sweden), which is comprised of recombinant Protein A ligands immobilised to a porous cross-linked agarose carrier.

The carrier to which ion-exchange chromatography ligands have been immobilised may be any of the kinds discussed above in relation to the affinity step. Thus, in one embodiment, the carrier is comprised of porous cross-linked polysaccharide particles to which ligands that exhibit charged groups have been immobilised. In one embodiment, the particles are essentially spherical and in a range from about 1 µm-1000 µm in diameter, such as 10-500 µm, such as about 90 µm. Said charged ligands may be positively charged, in case of an anion-exchanger, or negatively charged, in case of a cation-exchanger.

In one embodiment, the ion-exchange chromatography ligands have been immobilised to a carrier via extenders. Suitable extenders should be hydrophilic and may contain a plurality of groups selected, for instance, among hydroxy, carboxy, amino, repetitive ethylene oxide (-CH₂CH₂O-), amido etc. The extender may be in the form of a polymer such as a homo- or a copolymer. Hydrophilic polymeric extenders may be synthetic or natural polymers. Typical synthetic polymers are polyvinyl alcohols, polyacryl- and polymethacrylamides, polyvinyl ethers etc. Typical natural polymers are polysaccharides, such as starch, cellulose, dextran, and agarose. The preferred polymeric extenders are often water-soluble in their free state, i.e. when they are not attached to the carrier. Water-insoluble polymers or polymers of low original hydrophilicity may be made more hydrophilic by introducing hydrophilic groups therein. The size of the extender will depend on several factors, such as number of attachment points to the carrier, the chemical nature of the extender, the structure and size of the ligand, substitution degree, crosslinking degree etc. The flexibility of the coating or layer formed by the extender on the carrier will be increased for a decrease in the number of attachment points to the base matrix per extender molecule and/or in the degree of crosslinking of the extender. Extenders enabling one-point attachment, for instance at a terminal monomeric unit, may be small. For polymeric extenders for which attachment and/or crosslinking is possible at several monomeric units, i.e. tethered extenders, a larger size may be preferred. In one embodiment, the extenders are comprised of at least 30 monomeric units each, which for polysaccharides like dextran indicates a MW>5000 Da.

In order to control the flexibility of the extender, it is often advantageous to first activate the base matrix and then link the extender to the matrix by reaction with the activated groups introduced. Typical activation reagents are bifunctional in the sense that they are able to react twice with electrophilic functional groups. Illustrative examples are epihalohydrins, bisepoxides, CNBr etc. Other bifunctional reagents require an intermediary activation reaction, for instance allyl glycidyl ether and styryl glycidyl ether. For the latter reagents the first reaction takes place at the oxirane group, whereafter activation is caused by addition of halogen to the carbon-carbon double bond. The preferred bifunctional reagents should give rise to bridges that are stable against hydrolysis in the pH interval used in liquid chromatography. (For further details regarding extenders, see e.g. WO 98/33572 (Amersham Pharmacia Biotech, Sweden). The extenders may alternatively be grafted to the carrier e.g. as described in the above discussed WO 03/046063, Amersham Biosciences AB (Ihre et al)).

In the most preferred embodiment, said extenders have been tethered to the carrier, i.e. the extenders exhibit in average more than one contact point each with the carrier. In and advantageous embodiment, the extenders are comprised of a coating of dextran tethered to the carrier. Thus, this embodiment utilises an ion-exchange chromatography matrix which differs substantially from the one tested in the above discussed Chavez et al: "Increased Dynamic...", which used either Fractogel™ or Sepharose™. As mentioned above, Fractogel™ comprises ion-exchanger groups bonded to a carrier via linear polymer chains, and consequently will exhibit a much more flexible structure than a tethered extender. SP and Q Sepharose™ on the other hand are agarose carriers to which the ligands have been coupled via conventional spacers. Such spacers are substantially smaller molecules than extenders, and usually simply comprise the residues provided via the introduced activation and/or coupling agent.

In an illustrative embodiment, the ion-exchange chromatography step of the present method utilises SP or Q Sepharose™ XL (Amersham Biosciences, Uppsala, Sweden), which are comprised of cation exchanging ligands and anion exchanging ligands, respectively, immobilised to cross-linked agarose carriers via dextran extenders.

In a specific aspect, the present invention relates to a method of isolating an antibody or antibody compound from other component(s) of a liquid, which method comprises at least one chromatographic step, wherein in one step, the liquid is contacted with an ion-exchange chromatography matrix to adsorb the antibody compound in the presence of a buffer comprising a non-ionic polyether and at a conductivity which is equivalent to at least 200 mM NaAc. The buffer comprising the non-ionic polyether and the nature of the ion-exchange chromatography matrix are preferably as described above.

The carrier to which hydrophobic ligands have been immobilised may be any of the kinds discussed above in relation to the affinity step. In the preferred embodiment, the carrier is either made from a synthetic polymer, which is essentially hydrophobic as such, or from a carbohydrate material which has been made hydrophobic by modification of its hydrophilic groups, such as hydroxy groups. The hydrophobic groups of the present HIC matrix may be any well known kind, such as hydrophobic alkyl chains, aromatic groups etc. In one embodiment, the particles are essentially spherical and in a range from about 1 µm-1000 µm in diameter, such as 5-500 µm, preferably 5-25 µm, such as about 15 µm.

In addition, the present invention relates to the use of a non-ionic polyether, such as PEG, to the mobile phase of any series of two or more chromatographic steps. Such steps may be performed as conventional chromatography, i.e. by passing the mobile phase through a column packed with a separation matrix as described herein, or by expanded bed adsorption (EBA), wherein a fluidised bed is utilised. Thus, in addition to the above, the present invention may also or alternatively comprise a step of immobilised metal affinity chromatography (IMAC) and/or thiophilic chromatography, both of which are well known principles of chromatographic separation.

As mentioned above, the present method is useful to purify biological compounds such as proteins, e.g. antibodies and antibody compounds, peptides and nucleic acids from one or more other components of a liquid. In a specific embodiment, the present method is used to purify a liquid from one or more target compounds, i.e. the desired product is a liquid free from said compound(s). In one embodiment of the present method, the target compound is a protein. Examples of proteins are e.g. human serum albumin (HSA), ovalbumin, and lactoferrin. In an advantageous embodiment, the target compound is an antibody, such as a monoclonal or a polyclonal antibody, or a functional fragment of such an antibody. The antibody may be from any mammal, such as a human, or it may be a humanised antibody. The antibody purified using the present method may be selected from the group that consists of IgG, IgA, IgM, IgD and IgE.

Further, as is well known, therapeutic proteins and existing FDA-approved proteins are these days often modified with compounds that enhance their physical properties, such as solubility, hydrolytic stability and aggregation, as well as their biomedical properties, such as antigenicity, proteolytic stability, serum circulation time, and ease of delivery. At present, modification with poly(ethylene glycol) (PEG), commonly known as pegylation, is the most widely used for therapeutic applications. However, other compounds, such as PEG derivatives and neutral hydrophilic polymers, e.g. dextran, are alternatively used for such modification. The same kind of modification is also applied to other molecules than proteins, such as low molecular weight organic drugs and drug candidates. Thus, in a specific embodiment, the target compound is a compound, which has been modified as described above, preferably with poly(ethylene glycol) (PEG). Such compounds are commonly known as pegylated compounds. Pegylated compounds obtained according to the present invention are advantageously used in medicine, such as personalised medicine where a protein or non-protein drug is designed to cure a specific individual. Alternatively, the isolation according to the invention may be used for diagnostic purposes, such as to diagnose an individual by screening his or her blood for the presence of certain biological compounds or to determine quantitatively the amount of a specific biological compound.

A kit may be provided, said kit comprising at least two chromatography columns, each one packed with a matrix selected from the group that consists of affinity chromatography matrices, ion-exchange chromatography matrices and hydrophobic interaction chromatography (HIC) matrices; a buffer comprising a non-ionic polyether for addition to the mobile phase; and written instructions for its use. In a specific embodiment, the column is provided with luer adaptors, tubing connectors, and domed nuts. The columns may be of any well-known kind and material, and their volume may be adapted to laboratory scale or to production scale, i.e. small scale or large scale. The columns are prepacked with any two of the above mentioned kind of matrices. Further details to such matrices may be as presented above in the context of the first aspect of the invention. The non-ionic polyether may be as discussed above in relation to the first aspect of the invention. In the most advantageous embodiment, the non-ionic polyether is poly(ethylene glycol) (PEG). The instructions provided in the kit may explain any one of the above-described embodiments of the method according to the invention.

Finally, the present invention relates to a method of isolating an antibody compound from other component(s) of a liquid, which method comprises at least one chromatographic step, wherein in one step, the liquid is contacted with an ion-exchange chromatography matrix to adsorb the antibody compound in the presence of a buffer comprising a non-ionic polyether, wherein the ligands of the ion-exchange chromatography matrix have been immobilised to one or more porous carriers via dextran extenders. The nature of the ion-exchange chromatography matrix and the polyether may be as described above. Thus, in an advantageous embodiment, the non-ionic polyether is poly(ethylene glycol) (PEG). In one embodiment, the carriers are comprised of cross-linked polysaccharide particles. A kit for isolation of antibodies may be provided; which kit comprises, in separate compartments, an ion-exchange chromatography matrix wherein the ligands have been immobilised to porous carriers via dextran extenders, a buffer comprising poly(ethylene glycol) (PEG); and written instructions for adsorption of antibodies to the matrix. The kit is useful in the above described method.

### Detailed description of the drawings

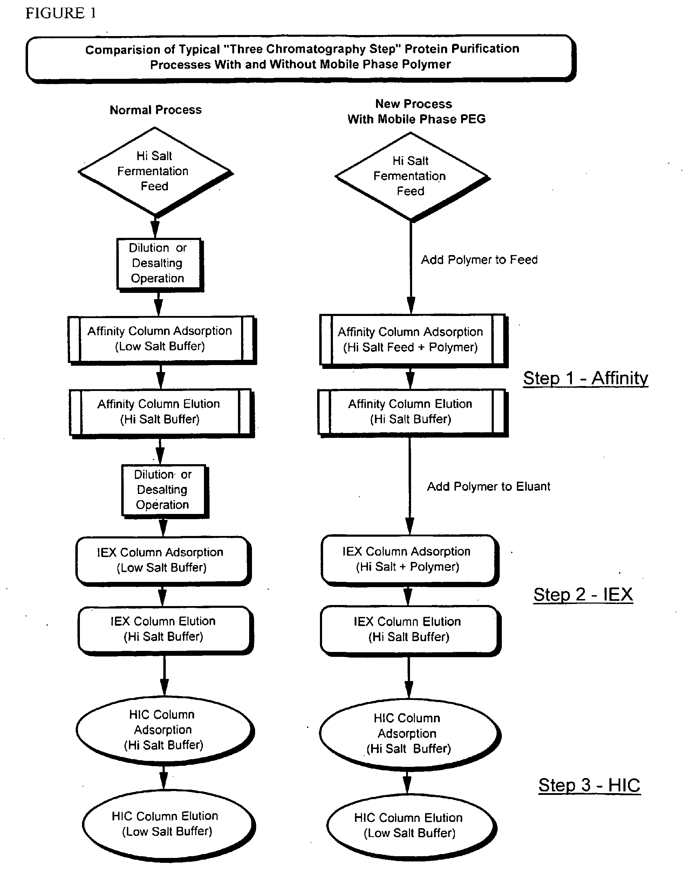

Figure 1 compares a three-step protein purification process according to the invention in the presence (right) and absence (left) of a non-ionic polyether, in this case PEG. More specifically, the illustrating process comprises the steps of affinity chromatography, ion-exchange chromatography and hydrophobic interaction chromatography. It appears clearly how the invention enables to reduce the number of unit operations, e. g. buffer dilution/desalting, necessary to interface the chromatographic steps.

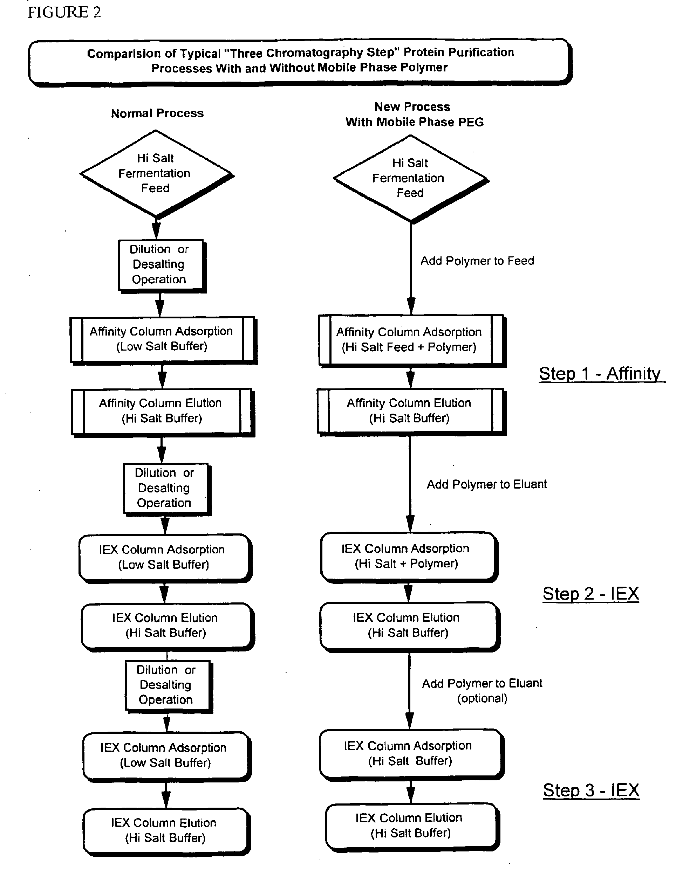

Figure 2 compares a three-step protein purification process according to the invention in the presence (right) and absence (left) of a non-ionic polyether, in this case PEG. More specifically, the illustrating process comprises the steps of affinity chromatography, ion-exchange chromatography and ion-exchange chromatography. Again, it appears clearly how the invention enables to reduce the number of unit operations, e. g. buffer dilution/desalting, necessary to interface the chromatographic steps.

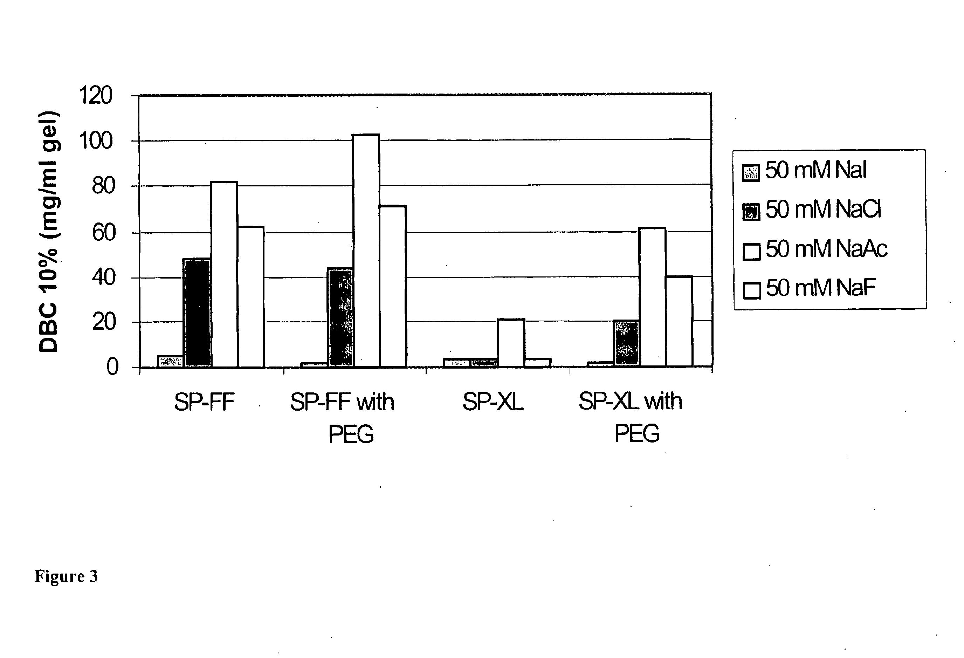

Figure 3 shows the effect of 8% PEG 10000 at increasing dynamic capacity of two commercial ion-exchange chromatography matrices, namely SP Sepharose™ FF (Amersham Biosciences, Uppsala, Sweden) and, to a greater extent, SP Sepharose™ XL (Amersham Biosciences, Uppsala, Sweden), which comprises dextran extenders, for bovine serum albumin (BSA). The results obtained are compared to the expected ion-exchange effect of different lyotropic buffer salts.

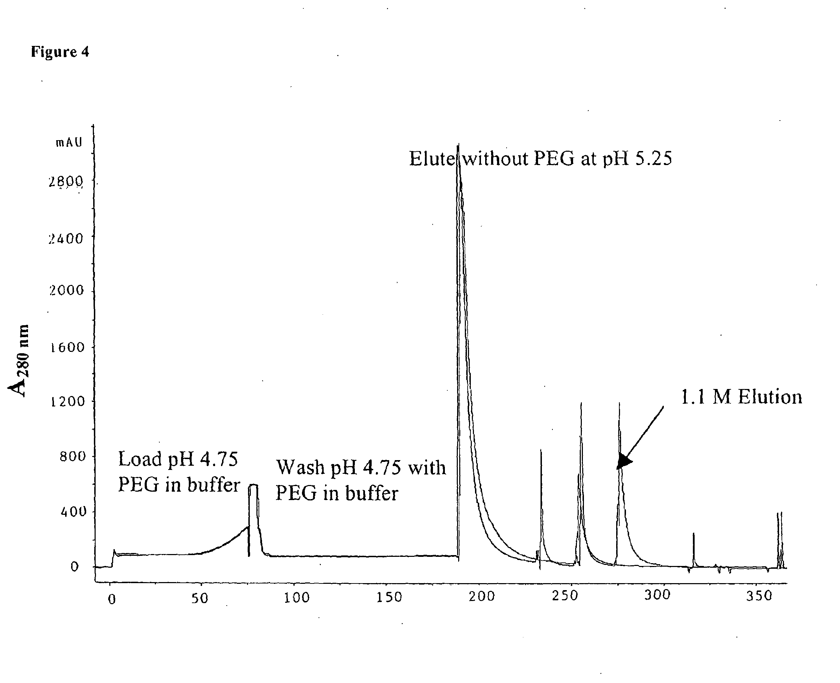

Figure 4 is a chromatogram showing SP Sepharose™ XL frontal analysis (chromatographic loading and elution) using BSA with NaAcetate pH 4.75 buffer plus 8% PEG 10000 protein loading, washing in same protein-free solution with protein retention on column, and elution in same buffer at pH 5.25 and without PEG. Raising elution buffer salt concentration to 1.1M does not enhance recovery.

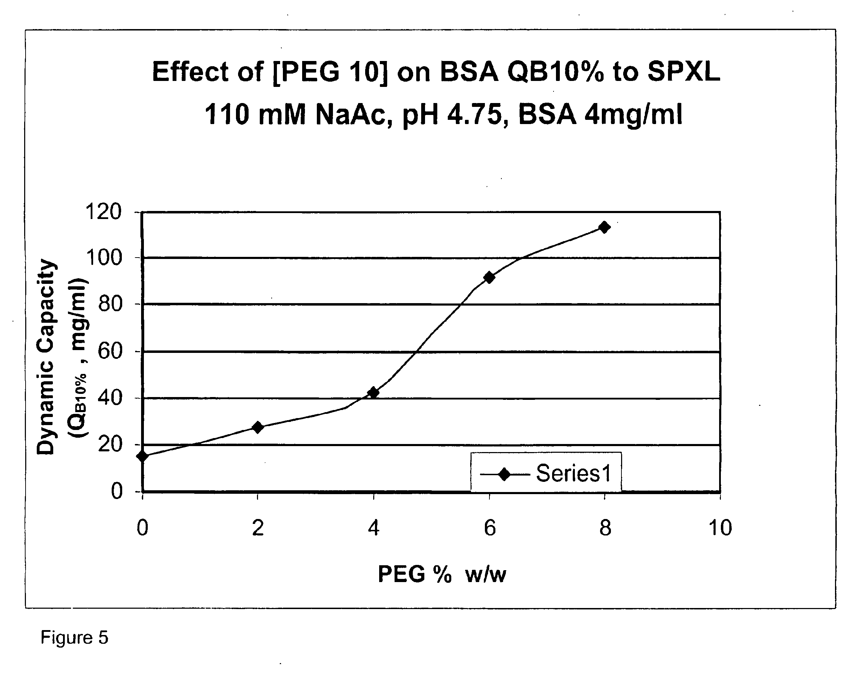

Figure 5 shows in a diagram how the BSA dynamic capacity of SP Sepharose™ XL matrix for BSA in 110 mM NaAcetate pH 4.75 increases with added mobile phase concentration (addition at adsorption) of PEG 10000.

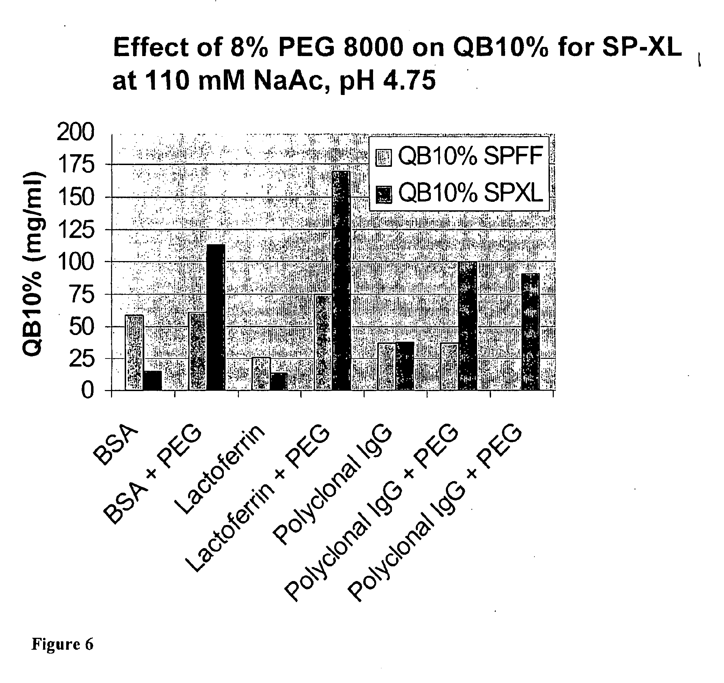

Figure 6 shows how mobile phase added PEG increases the dynamic capacity of SP Sepharose™ FF and SP Sepharose™ XL for various proteins including BSA and lactoferrin, which in the absence of PEG show reduced dynamic capacity in the SP Sepharose™ XL versus SP Sepharose™ FF matrix.

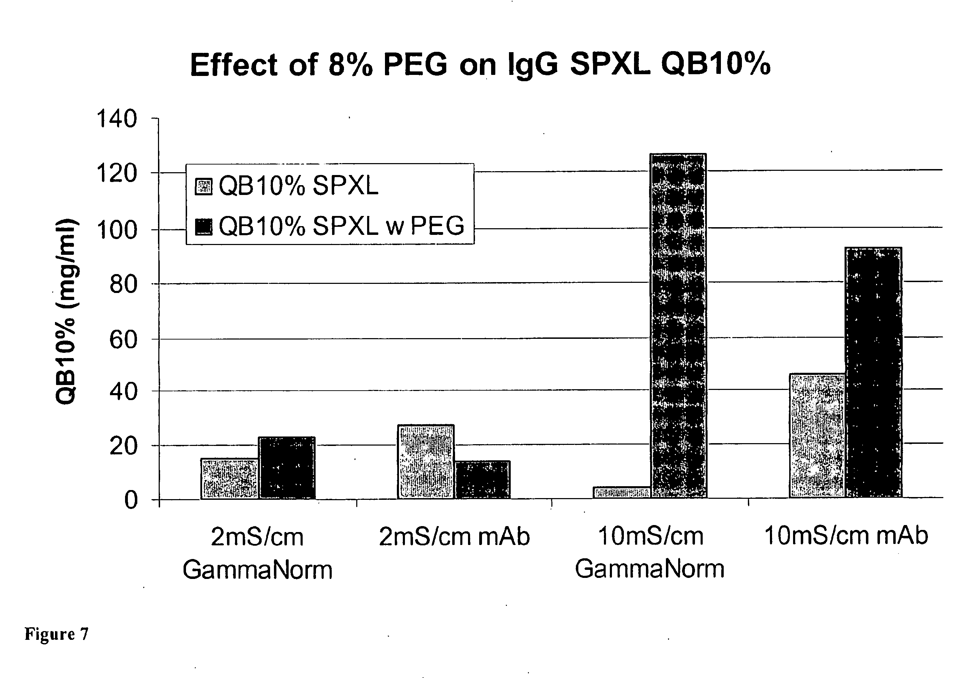

Figure 7 shows how mobile phase added PEG enhances the dynamic capacity of SP Sepharose™ XL matrix for both polyclonal and monoclonal antibody samples, with the effect more pronounced in buffers with higher salt concentration.

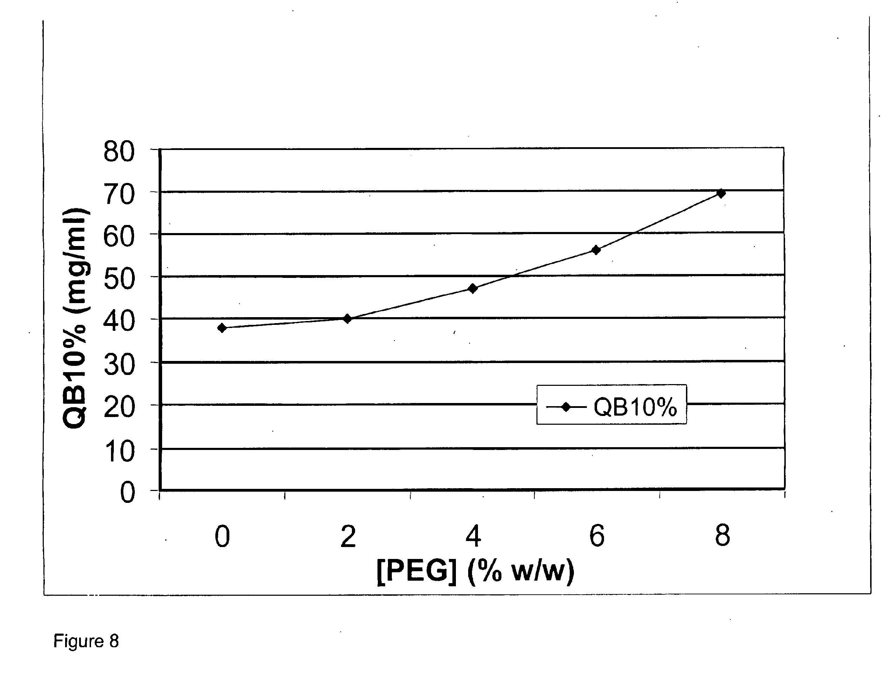

Figure 8 shows how mobile phase added PEG is able to also enhance the dynamic capacity of the commercially available affinity chromatography matrix MabSelect™, which comprises Protein A ligands attached to a carrier of cross-linked agarose (Amersham Biosciences, Uppsala, Sweden) for polyclonal antibody.

### EXPERIMENTAL PART

The present examples are provided for illustrative purposes only, and should not be construed as limiting the scope of the appended claims.

### Example 1

In this example, a typical determination of dynamic capacity using frontal analysis was performed according to well known principles, as disclosed e.g. in Protein Purification - Principles, High Resolution Methods and Applications (J.-C. Janson and L. Rydén, 1989 VCH Publishers, Inc.). Studies were performed on ÄKTA™ chromatography work station (Amersham Biosciences, Uppsala, Sweden) using standard HR 5/10 or 5/5 columns (Amersham Biosciences, Uppsala, Sweden) packed with 1ml or 2 ml, respectively, of the cation-exchange chromatography matrices SP Sepharose™ XL™ or SP Sepharose™ FF (Amersham Biosciences, Uppsala, Sweden), buffer salts and protein (bovine serum albumin, BSA) from Sigma-Aldrich. The buffer solutions were all at room temperature and 50 mM salt adjusted to pH 4.75. BSA in solution at 4 mg/ml was slowly (<1 ml/min, see below) pumped through the column, which adsorbed the protein until such time as the column reached its dynamic capacity. At this point UV A280 nm of solution coming off the column started to increase. Taking the volume at which this A280 reaches 10% of the value of the 4 mg/ml protein loading solution and multiplying by the amount of solution pumped through the column, then dividing by the column bed volume indicates the amount of protein on the column (mg/ml packed matrix) or Q_{B10%}. This value can also be checked against the amount of protein recovered from the column when it is washed with buffer at higher salt concentration (e. g. 0.5 M) and/or at pH close to that of the protein (e. g. 5.3 for BSA). Such a comparison indicates adsorbed versus recovered protein. In the present experiment, the frontal analysis was run using normal buffer (noted salt plus 5 mM NaAc with pH adjusted to 4.75) with and without 8% (w/w) added PEG 10000 (Fluka), which readily dissolves in the buffer. Generally speaking for ion-exchange chromatography one might expect Q_{B10%} to increase as the initial buffer salt changes in the lyotropic order NaI to NaCl to NaAc to NaF. Such results are shown for SP Sepharose™ FF (SPFF) and SP Sepharose™ XL (SPXL) matrix in Figure 3 under conditions with and without addition of 8% PEG. The figure also indicates that (a) under these normal, low IEX adsorption salt concentration, i.e. 50 mM, conditions the BSA dynamic capacity of SPFF exceeds that of SPXL, and (b) addition of PEG slightly increases SPFF dynamic capacity but greatly increases SPXL capacity to a more useful dynamic capacity. See also Figure 4.

### Example 2

In this example, simple BSA frontal analysis data were obtained for SPXL matrix (as described above for Figure 3) run in 5/10 column with protein being initially adsorbed from 0.22M NaAcetate buffer pH 4.75 containing 8% PEG 10000 (Fluka). This is 4x the normal IEX adsorption salt concentration, e. g. 0.05M, see example 1 above. The results are shown in Figure 4. At 10% breakthrough, the column is washed with the same buffer containing PEG to demonstrate that protein does not wash off the column. The protein on the column is then eluted using similar 0.22M NaAcetate buffer at pH 5.25 (closer to the pI of BSA) without added PEG. The protein is seen to elute in a sharp peak with total of 180 mg protein or 90 mg/ml SPXL, to be compared with Figure 3 and values of 20 and 60 mg/ml for BSA on SPXL at 0.05M NaAcetate. Following the same process but eluting with 1.1M buffer does not significantly enhance protein dynamic capacity.

### Example 3

In this example, the effect of increasing mobile phase added PEG 10000 (Fluka) on the dynamic capacity data of SPXL matrix for BSA obtained as described above was studies, but with adsorption using 110 mM NaAcetate buffer at pH 4.75. The results are shown in Figure 5. As can be expected, at PEG concentrations approaching 10% the relatively high salt concentration caused solution clouding and protein precipitation.

### Example 4

In this example, 8% w/w PEG 8000 (Sigma) was added to the mobile phase and the SPXL dynamic binding capacities of several proteins was determined using the procedures similar to those described above for Figures 1 to 3. More specifically, the proteins were BSA and lactoferrin from Sigma-Aldrich, and GammaNorm™ polyclonal human IgG from OctaFarma. The results are shown in Figure 6. The adsorption buffer was 110 mM NaAcetate pH 4.75 with and without 8% added PEG 8000 (Sigma). It should be noted that under these conditions of relatively high salt concentration, both lactoferrin and BSA normally show both reduced and poor dynamic capacity in SPXL compared to SPFF matrix. However addition of PEG to the mobile phase greatly enhanced the dynamic capacity of these proteins as well as that of monoclonal human antibody.

### Example 5

In this example, the effect of added 8% w/w PEG 8000 (Sigma) on SPXL dynamic binding capacities of several proteins were determined using the procedures similar to those described above for Figures 3 and 4. More specifically, the proteins were BSA from Sigma-Aldrich, GammaNorm™ polyclonal human IgG preparation from OctaFarma, and a research lab monoclonal antibody sample from Amersham Biosciences. The results are shown in Figure 7. HR 5/5 columns packed with 1 ml of SPXL were run at 0.33 ml/min. Adsorption buffers were 15 mM NaAcetate pH 5 (approx 2mS/cm) and 15mM NaAcetate pH 5 with added 200 mM NaCl to reach 10 mS/cm.

### Example 6

In this example, the effect of mobile phase added PEG (0 to 8% w/w PEG 10000 from Fluka) on the dynamic capacity of an affinity chromatography matrix, namely MabSelect™ (Amersham Biosciences, Uppsala which comprises protein A ligands, for polyclonal human antibody was studied. The results are shown in Figure 8. The matrix was a MabSelect research sample (U631029A of slightly higher than normal protein A content) from Amersham Biosciences and the protein was GammaNorm from OctaFarma. Approximately 2ml of matrix was packed in HR 5/10 column (Amersham Biosciences) and run at 240 cm/h with 2.4 min residence time using buffer containing 150 mM NaCl and 20 mM NaPhosphate pH 7.4. Protein was eluted in 30 mM NaCitrate pH 3 solution and columns were cleaned of residual protein using 0.01M NaOH solution. Similar results with slightly lower dynamic capacity values (i.e. 43 mg/ml with 6% PEG) were obtained using commercial MabSelect matrix (not shown). In addition gel filtration studies done under the above conditions suggested the apparent increase in dynamic capacity was not due to protein-protein interactions such as aggregation (results not shown) and that the PEG could be added up to 6% to real fermentation feed (not shown).

## Claims

1. A method of isolating one or more target compounds from other component(s) of a liquid by at least two chromatographic steps, which method comprises contacting the liquid, in any sequence of order, with an affinity chromatography matrix and an ion-exchange chromatography matrix and/or a hydrophobic interaction chromatography matrix to provide interactions between the target compound and the matrices; and obtaining the target compound(s) in a separate fraction from the last chromatographic step, **characterized in that** the contacting with the affinity chromatography matrix takes place in the presence of at least one non-ionic polyether.

2. A method according to claim 1, wherein the target compound(s) are adsorbed to one or more of the chromatography matrices.

3. A method according to claim 2, wherein the adsorbed target compound(s) are released by contacting the chromatography matrix with an eluent.

4. A method according to any one of the preceding claims, which comprises two or more consecutive ion-exchange chromatography steps.

5. A method according to any one of claims 1-3, which comprises an affinity step followed by an ion-exchange chromatography step.

6. A method according to any one of claims, 1-3, which comprises an ion-exchange chromatography step followed by a hydrophobic interaction chromatography step.

7. A method according to any one of the preceding claims, which comprises three chromatographic steps.

8. A method according to any one of the preceding claims, wherein the method comprises affinity chromatography followed by ion exchange chromatography and/or hydrophobic interaction chromatography.

9. A method according to any one of the preceding claims, wherein at least two steps are performed in the presence of a non-ionic polyether.

10. A method according to any one of the preceding claims, wherein the non-ionic polyether is poly(ethylene glycol) (PEG).

11. A method according to any one of the preceding claims, wherein the target compound is an antibody or an antibody compound.

12. A method according to any one of the preceding claims, which comprises an affinity step using a matrix comprised of protein ligands immobilised to porous carriers.

13. A method according to claim 12, wherein the protein ligands comprises one or more of the immunoglobulin-binding domains of Protein A.

14. A method according to claim 12 or 13, wherein the carriers are comprised of cross-linked polysaccharide particles.

15. A method according to any one of the preceding claims, which comprises an ion-exchange step using a matrix comprised of ligands comprising charged groups, which ligands have been immobilised to a carrier via extenders.

16. A method according to claim 15, wherein the extenders are provided by coating the carrier surfaces with dextran.

17. A method according to claim 15 or 16, wherein the carriers are comprised of porous cross-linked polysaccharide particles.

18. A method according to any one of the preceding claims, wherein the liquid is contacted with an ion-exchange chromatography matrix to adsorb an antibody compound in the presence of a buffer comprising a non-ionic polyether and at a conductivity which is equivalent to at least 200 mM NaAc.

## Patentansprüche

1. Verfahren zur Isolierung einer oder mehrerer Zielverbindungen aus (einer) anderen Komponente(n) einer Flüssigkeit durch mindestens zwei chromatographische Schritte, das Verfahren umfassend das Kontaktieren der Flüssigkeit in jeder möglichen Reihenfolge mit einer Affinitätschromatographie-Matrix und einer lonenaustauschchromatographie-Matrix und/oder einer hydrophoben Wechselwirkungschromatographie-Matrix, um Wechselwirkungen zwischen der Zielverbindung und den Matrizes zur Verfügung zu stellen, sowie das Erhalten der Zielverbindung(en) in einer separaten Fraktion aus dem letzten chromatographischen Schritt, **dadurch gekennzeichnet, dass** das Kontaktieren mit der Affinitäts-Matrix in Gegenwart mindestens eines nicht-ionischen Polyethers erfolgt.

2. Verfahren nach Anspruch 1, wobei die Zielverbindung(en) auf einer oder mehrer der Chromatographie-Matrizes adsorbiert werden.

3. Verfahren nach Anspruch 2, wobei die adsorbierte(n) Zielverbindung(en) durch Kontaktieren der Chromatographie-Matrix mit einem Eluenten freigesetzt wird/werden.

4. Verfahren nach einem der vorhergehenden Ansprüche, welches zwei oder mehr aufeinanderfolgende Ionenaustauschchromatographie-Schritte umfasst.

5. Verfahren nach einem der Ansprüche 1 bis 3, welches einen Affinitätsschritt gefolgt von einem lonenaustauschchromatographie-Schritt umfasst.

6. Verfahren nach einem der Ansprüche 1 bis 3, welches einen lonenaustauschchromatographie-Schritt gefolgt von einem hydrophoben Wechselwirkungschromatographie-Schritt umfasst.

7. Verfahren nach einem der vorhergehenden Ansprüche, welches drei Chromatographieschritte umfasst.

8. Verfahren nach einem der vorhergehenden Ansprüche, wobei das Verfahren Affinitätschromatographie gefolgt von lonenaustausch-Chromatographie und/oder hydrophober Wechselwirkungschromatographie umfasst.

9. Verfahren nach einem der vorhergehenden Ansprüche, wobei mindestens zwei Schritte in Gegenwart eines nicht-ionischen Polyethers durchgeführt werden.

10. Verfahren nach einem der vorhergehenden Ansprüche, wobei der nichtionische Polyether Polyethylenglykol (PEG) ist.

11. Verfahren nach einem der vorhergehenden Ansprüche, wobei die Zielverbindung ein Antikörper oder eine Antikörper-Verbindung ist.

12. Verfahren nach einem der vorhergehenden Ansprüche, welches einen Affinitätsschritt unter Verwendung einer Matrix, welche einen porösen Träger immobilisierter Proteinliganden beinhaltet, umfasst.

13. Verfahren nach Anspruch 12, wobei die Proteinliganden eine oder mehrere der Immunoglobulin-bindenden Domänen von Protein A umfassen.

14. Verfahren nach Anspruch 12 oder 13, wobei die Träger quervernetzte Polysaccharid-Partikel beinhalten.

15. Verfahren nach einem der vorhergehenden Ansprüche, welches einen lonenaustausch-Schritt unter Verwendung einer Matrix beinhaltend Liganden, welche geladene Gruppen beinhalten, wobei die Liganden über Füllstoffe an einem Träger immobilisiert worden sind, umfasst.

16. Verfahren nach Anspruch 15, wobei die Füllstoffe durch Beschichten der Trägeroberflächen mit Dextran bereitgestellt werden.

17. Verfahren nach Anspruch 15 oder 16, wobei die Träger poröse, quervernetzte Polysaccharid-Partikel umfassen.

18. Verfahren nach einem der vorhergehenden Ansprüche, wobei die Flüssigkeit mit einer lonenaustauschchromatographie-Matrix kontaktiert wird, um eine Antikörper-Verbindung in Gegenwart eines Puffers beinhaltend einen nicht-ionischen Polyether bei einer Leitfähigkeit, welche zu mindestens 200 mM NaAc äquivalent ist, adsorbiert.

## Revendications

1. Procédé d'isolement d'un ou plusieurs composés cibles par rapport à un ou d'autres composants d'un liquide par au moins deux étapes de chromatographie, lequel procédé comprend la mise en contact du liquide, suivant une séquence d'ordre quelconque, avec une matrice de chromatographie d'affinité et une matrice de chromatographie d'échange d'ion et/ou une matrice de chromatographie d'interaction hydrophobe afin de produire des interactions entre le composé cible et les matrices ; et l'obtention du ou des composés cibles dans une fraction séparée à partir de la dernière étape de chromatographie, **caractérisé en ce que** la mise en contact avec la matrice de chromatographie d'affinité est réalisée en la présence d'au moins un polyéther non ionique.

2. Procédé selon la revendication 1, dans lequel les composés cibles sont adsorbés sur une ou plusieurs des matrices de chromatographie.

3. Procédé selon la revendication 2, dans lequel le ou les composés cibles adsorbés sont libérés par mise en contact de la matrice de chromatographie avec un éluant.

4. Procédé selon l'une quelconque des revendications précédentes, qui comprend deux ou plusieurs étapes de chromatographie d'échange d'ion consécutives.

5. Procédé selon l'une quelconque des revendications 1 à 3, qui comprend une étape par affinité suivie par une étape de chromatographie d'échange d'ion.

6. Procédé selon l'une quelconque des revendications 1 à 3, qui comprend une étape de chromatographie d'échange d'ion suivie par une étape de chromatographie d'interaction hydrophobe.

7. Procédé selon l'une quelconque des revendication précédentes, qui comprend trois étapes de chromatographie.

8. Procédé selon l'une quelconque des revendications précédentes, dans lequel le procédé comprend une chromatographie d'affinité suivie par une chromatographie d'échange d'ion et/ou une chromatographie d'interaction hydrophobe.

9. Procédé selon l'une quelconque des revendications précédentes, dans lequel au moins deux étapes sont mises en oeuvre en la présence d'un polyéther non ionique.

10. Procédé selon l'une quelconque des revendications précédentes, dans lequel le polyéther non ionique est du poly(éthylène glycol) (PEG).

11. Procédé selon l'une quelconque des revendications précédentes, dans lequel le composé cible est un anticorps ou un composé d'anticorps.

12. Procédé selon l'une quelconque des revendications précédentes, qui comprend une étape par affinité en utilisant une matrice constituée par des ligands de protéine immobilisés sur des supports poreux.

13. Procédé selon la revendication 12, dans lequel les ligands de protéine comprennent un ou plusieurs des domaines de liaison d'immunoglobuline de la protéine A.

14. Procédé selon la revendication 12 ou 13, dans lequel les supports sont constitués par des particules polysaccharides réticulées.

15. Procédé selon l'une quelconque des revendications précédentes, qui comprend une étape par échange d'ion en utilisant une matrice constituée par des ligands comprenant des groupes chargés, lesquels ligands ont été immobilisés sur un support par l'intermédiaire d'agents expanseurs.

16. Procédé selon la revendication 15, dans lequel les agents expanseurs sont produits par revêtement des surfaces supports avec du dextrane.

17. Procédé selon la revendication 15 ou 16, dans lequel les supports sont constitués par des particules polysaccharides réticulées poreuses.

18. Procédé selon l'une quelconque des revendications précédentes, dans lequel le liquide est mis en contact avec une matrice de chromatographie d'échange d'ion afin d'adsorber un composé d'anticorps en la présence d'un tampon comprenant un polyéther non ionique et à une conductivité qui est équivalente à celle d'au moins 200 mM de NaAc.
